# EUROPÄISCHE PATENTSCHRIFT

(11) **EP 3 669 899 B1**
(45) Veröffentlichungstag und Bekanntmachung des Hinweises auf die Patenterteilung: **17.08.2022**
(21) Anmeldenummer: 19215604.0
(22) Anmeldetag: 12.12.2019
(51) Int. Cl.: A61L 15/42, A61L 15/44, A61L 15/56, A61L 26/00

(54) **WUNDAUFLAGE MIT PARTIKELN, WELCHE EINEN FLUORESZENZFARBSTOFF FREISETZEN KÖNNEN**
WOUND DRESSING COMPRISING PARTICLES CAPABLE OF RELEASING FLUORESCENT DYE
PANSEMENT COMPORTANT DES PARTICULES POUVANT LIBÉRER UN COLORANT FLUORESCENT

(30) Priorität: 19.12.2018 DE 102018132884
(43) Veröffentlichungstag der Anmeldung: 24.06.2020
(73) Patentinhaber: Paul Hartmann AG, 89522 Heidenheim (DE)
(72) Erfinder: Kettel, Markus, 89520 Heidenheim (DE)

(56) Entgegenhaltungen:
- EP-A1- 2 361 502
- EP-A1- 3 643 331
- DE-T2- 60 126 506
- DE-T2- 60 203 403
- KIETZMANN D ET AL: "Colonic delivery of carboxyfluorescein by pH-sensitive microspheres in experimental colitis", EUROPEAN JOURNAL OF PHARMACEUTICS AND BIOPHARMACEUTICS, ELSEVIER SCIENCE PUBLISHERS B.V., AMSTERDAM, NL, Bd. 76, Nr. 2, 1. Oktober 2010 (2010-10-01), Seiten 290-295, XP027369227, ISSN: 0939-6411 [gefunden am 2010-07-01]
- YOO J W ET AL: "pH-sensitive Eudragit nanoparticles for mucosal drug delivery", INTERNATIONAL JOURNAL OF PHARMACEUTICS, ELSEVIER, NL, Bd. 403, Nr. 1-2, 17. Januar 2011 (2011-01-17), Seiten 262-267, XP027554474, ISSN: 0378-5173 [gefunden am 2010-10-31]
- DATABASE EPODOC [Online] EUROPEAN PATENT OFFICE, THE HAGUE, NL; XP002798926, Database accession no. CN-201711247171-A & CN 107 823 692 A (GUANGZHOU ZHONGWEI BIOTECHNOLOGY CO LTD ET AL.) 23. März 2018 (2018-03-23)

## Beschreibung

Die vorliegende Erfindung betrifft Wundauflagen, insbesondere zur Behandlung von chronischen und/oder infizierten Wunden.

Die Heilung von Hautwunden beruht auf der Fähigkeit der Haut, Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, inflammatorische Phase oder Reinigungsphase), die proliferative Phase zum Aufbau von Granulationsgewebe (Phase 2, Proliferations- oder Granulationsphase) und die Differenzierungsphase zur Epithelisierung und Narbenbildung (Phase 3, Differenzierungs- oder Epithelisierungsphase). Es hat sich gezeigt, dass eine Abheilung der Wunde durch eine moderne, feuchte Wundbehandlung besonders gefördert wird. Im Rahmen der feuchten Wundbehandlung werden unter anderem Wundauflagen mit einer Schaumschicht eingesetzt. Solch eine Schaumschicht bietet nachwachsendem Gewebe eine Matrix, die die Wundheilung stimuliert, und kann gleichzeitig größere Mengen Wundexsudat aufnehmen und binden. Außerdem hat es sich bewährt, für die Feuchthaltung von Wunden Hydrogele einzusetzen, die zudem Hautirritationen am Wundrand verringern.

Es ist seit Langem bekannt, dass es bei der Wundheilung, insbesondere während der Entzündungs- und der Granulationsphase der Wundheilung, zu Störungen kommen kann. Im Zusammenhang mit der vorliegenden Erfindung schließt der Begriff Wunde den Wundgrund ein. In der Wunde kann sich Wundexsudat befinden. Die Wundheilung kann dabei vom pH-Wert einer Wunde bzw. vom pH-Wert des in der Wunde enthaltenen Wundexsudats beeinflusst werden. Gesunde Haut weist üblicherweise einen pH-Wert im Sauren auf, der ungefähr zwischen pH 4,0 bis pH 5,7 liegt.

Bei Wunden, welche eine gestörte Wundheilung aufweisen, insbesondere bei chronischen oder infizierten Wunden, kann häufig ein alkalischer pH-Wert der Wunde oder des Wundexsudats beobachtet werden. Die Messung kann beispielsweise durch eine in-vitro-Messung des pH-Werts von Wundexsudat erfolgen. Eine Verschiebung des pH-Wertes vom Sauren ins Alkalische kann beispielsweise durch die Vermehrung von Bakterien oder durch die Bildung von Nekrosen verursacht werden.

Nekrosen und pathologische Mikroorganismen können auf den physiologischen Metabolismus während des Wundheilungsgeschehens einwirken. Dies führt häufig zu lokaler Hypoxie, und dann zum weiteren Abbau umliegenden Gewebes. Das entstehende alkalische Milieu kann weitere gewebeabbauende Prozesse begünstigen. Weiterhin kann das alkalische Milieu die Vermehrung weiterer pathogener Mikroorganismen stimulieren und so die Wundheilung zusätzlich behindern. Pathogene Keime können in einem gewissen Umfang durch die lokale topische Applikation antimikrobieller Therapeutika, insbesondere Antibiotika, bekämpft werden. Wünschenswert ist es hierbei, wenn Antibiotika nur dann appliziert werden, wenn ein hinreichender Verdacht auf eine mikrobielle Infektion vorliegt. Die rein prophylaktische Applikation von Antibiotika soll dagegen vermieden werden.

Chronische Wunden sind solche Wunden, deren Heilungsverlauf in einem oder allen Stadien der Wundheilung von der normalen Wundheilung abweicht. So kann aus akuten, normal heilenden Wunden z.B. durch eine Wundinfektion eine chronische Wunde entstehen, die durch eine verzögerte Heilungsgeschwindigkeit gekennzeichnet ist. Der Übergang von einer akuten zu einer chronischen Wunde kann dabei in jedem Stadium der Wundheilung erfolgen. Klinisch werden chronische Wunden definiert als Wunden, deren Heilung mehr als 6-8 Wochen benötigen, wobei diese Definition nicht alle Krankheitsbilder korrekt abdeckt. Es handelt sich bei chronischen Wunden mehr um eine Diagnose, die sich auf die klinische Erfahrung des medizinischen Personals stützt. Es wäre wünschenswert, dem medizinischen Personal bei der Diagnosestellung zusätzliche objektive Indikatoren in die Hand zu geben, so dass bei einem Verdacht auf eine ernsthafte Wundheilungsstörung schon möglichst frühzeitig geeignete therapeutische Maßnahmen ergriffen werden können.

Chronische Wunden entstehen insbesondere aufgrund einer mechanischen Belastung (Dekubitus, Druck-Ulzera, Druckgeschwür), einer venösen Insuffizienz (Ulcus cruris venosum, venöse Ulzera), einer artheriosklerotischen Gefäßveränderung (Ulcus cruris arteriosum, arterielle Ulzera), einer neuropathischen Veränderungen (diabetisches Fußsyndrom, neuropathische Ulzera), aber auch in Folge von Autoimmunerkrankungen, von Tumoren (exulzierende Tumore) oder Strahlenschäden bei der Tumortherapie.

WO02/47737 offenbart eine Wundauflage mit einem absorbierenden Hydrogel und einer durch eine Sperrschicht umschlossene zusätzliche absorbierende Schicht. Die Sperrschicht ist bei einem leicht alkalischen pH-Wert besser löslich als im neutralen Milieu. Anfallendes Wundexsudat wird zunächst von der Hydrogelschicht absorbiert. Bei einem Ansteigen des pH-Wertes löst sich die Sperrschicht auf, so dass Wundexsudat vom Hydrogel zur absorbierenden Schicht durchdringen kann. Es ist auch möglich, dass durch das Auflösen der Sperrschicht antimikrobielle Substanzen, die beispielsweise in der absorbierenden Schicht dispergiert sind, zur Wunde hin abgegeben werden.

Die WO 2013/036771 beschreibt pH-empfindliche Zusammensetzungen, die ein Antibiotikum oder ein Antibiotikum und einen pH-Indikator umfassen, sowie Wundverbände, die solche Zusammensetzungen enthalten. Die pH-empfindlichen Zusammensetzungen werden als weitgehend stabil unter basischem und neutralem pH-Wert beschrieben. Bei einem saurem pH-Wert werden die Schichten aufgrund von CO₂-Bildung abgebaut, so dass das darin enthaltene Antibiotikum freigesetzt werden kann. Weiterhin kann der Indikator bei einer pH-Änderung einen sichtbaren Farbumschlag zeigen. EP2361502 beschreibt Wundauflagen mit Nanokapseln in die antimikrobielle Mittel sowie Signalstoffen, insbesondere Farbstoffe wie zum Beispiel Fluoreszenzfarbstoffe, eingeschlossen sind.

Insgesamt stellte sich die Aufgabe, die Erkennung und Behandlung chronischer Wunden zu verbessern und die im Stand der Technik beschriebenen Nachteile zu überwinden. Hierzu wird die im Folgenden näher beschriebene Wundauflage vorgeschlagen. Ebenso werden zur Verwendung in der Diagnostik und Behandlung von Wunden verwendbare Partikel vorgeschlagen (nicht erfindungsgemäß). Wundauflage und Partikel sind in den Ansprüchen definiert. Die vorliegende Erfindung betrifft Wundauflagen enthaltend Partikel, welche ein pH-sensitives Polymer und einen Fluoreszenzfarbstoff umfassen, dadurch gekennzeichnet, dass die Löslichkeit oder Lösungsgeschwindigkeit des pH-sensitiven Polymers bei einem saurem pH-Wert geringer als bei einem neutralen oder alkalischem pH-Wert ist, weiter dadurch gekennzeichnet, dass die Partikel einen Durchmesser von 50 nm bis 1000 µm, bevorzugt 5 µm bis 300 µm, aufweisen.

Sofern die Partikel beim Einsatz einer erfindungsgemäßen Wundauflage in der Behandlung einer Wunde mit den Wundsekreten in Kontakt kommen, kann sich das pH-sensitive Polymer infolge einer Veränderung des pH-Wertes der Wunde auflösen und so einen in den Partikeln angereicherten Fluoreszenzfarbstoff freisetzen. Hierdurch kann die Fluoreszenzlöschung beim Fluoreszenzfarbstoff nachlassen und die Fluoreszenzintensität zunehmen. Eine von außen nachweisbare Veränderung der Fluoreszenzintensität deutet somit mittelbar auf eine Veränderung in der Wunde hin. Insbesondere ist es wünschenswert, eine Veränderung des pH-Wertes, insbesondere einen Anstieg des pH-Wertes während der Behandlung frühzeitig erkennen zu können, ohne dass die Wundauflage von der Wunde entfernt werden muss.

Im Rahmen der vorliegenden Erfindung wird unter einer Wundauflage ein Gegenstand verstanden, welcher auf einer Wundoberfläche an einem Patienten angebracht wird. Bei einer erfindungsgemäßen Wundauflage kann es sich um eine primäre Wundauflage handeln, also um eine Wundauflage, die direkt auf der zu behandelnden Wunde aufgebracht wird, oder um eine sekundäre Wundauflage, die über einer getrennt davon applizierten Wundauflage auf die Wunde gebracht wird. Eine geeignete Wundauflage kann eine einzelne Wundkontaktschicht umfassen oder aus mehreren Schichten zusammengesetzt sein. Diese Schichten können unterschiedliche Funktionen aufweisen.

Erfindungsgemäß enthält die Wundauflage Partikel, welche ein pH-sensitives Polymer und einen Fluoreszenzfarbstoff umfassen, dadurch gekennzeichnet, dass die Löslichkeit oder Lösungsgeschwindigkeit des pH-sensitiven Polymers bei einem saurem pH-Wert geringer als bei einem neutralen oder alkalischem pH-Wert ist, weiter dadurch gekennzeichnet, dass die Partikel einen Durchmesser von 50 nm bis 1000 µm, bevorzugt 5 µm bis 300 µm, aufweisen.

Erfindungsgemäß ist vorgesehen, dass die Wundauflage hierbei eine Vielzahl von Partikeln enthält, beispielsweise mehr als 10 Partikel pro cm².

Die Partikel können in verschiedenen Lagen einer Wundauflage untergebracht werden, ohne dass die physikalischen Eigenschaften der jeweiligen Lage beeinträchtigt werden, wie beispielsweise Absorptionsvermögen, Elastizitätsmodul oder Quellvermögen.

Im Rahmen der vorliegenden Anmeldung werden unter dem Begriff "Partikel" alle Partikel verstanden, ungeachtet ihrer Gestalt, deren Äquivalentdurchmesser im Größenordnungsbereich von 50 nm bis 1000 µm liegt (derartige Partikel werden allgemein auch als Mikropartikel bezeichnet).

Dabei bezieht sich der Unterbegriff Mikrosphärule auf sphärisch geformte Partikel, während der Ausdruck Mikrokapsel solche Partikel beschreibt, bei denen eine äußere Polymerschicht einen flüssigen, gasförmigen oder festen Kern umhüllt. Partikel, in denen eine Substanz, beispielsweise der Fluoreszenzfarbstoff, gleichmäßig mehr oder weniger fein im Polymer verteilt vorliegt, bezeichnet man auch als Mikromatrices.

Bevorzugt handelt es sich bei den verwendeten Partikeln um Mikromatrices oder Mikrokapseln.

Die Partikel weisen einen Durchmesser von 50 nm bis 1000 µm, besonders bevorzugt 5 µm bis 300 µm, auf.

Im Rahmen der vorliegenden Erfindung wird unter einem pH-sensitiven Polymer eine Substanz oder ein Substanzgemisch verstanden, welches aus einer Vielzahl sich wiederholender Monomereinheiten aufgebaut ist und das in einer wässrigen Lösung bei einer bestimmten Temperatur und einem ersten pH-Wert eine erste Löslichkeit bzw. Lösungsgeschwindigkeit aufweist und bei derselben Temperatur und einem zweiten pH-Wert, der vom ersten pH-Wert verschieden ist, eine zweite Löslichkeit bzw. Lösungsgeschwindigkeit, die von der ersten Löslichkeit bzw. Lösungsgeschwindigkeit verschieden ist. Daraus ergeben sich unterschiedliche Löslichkeiten bzw. Lösungsgeschwindigkeiten des Polymers bei verschiedenen pH-Werten.

Hierbei ist erfindungsgemäß die Löslichkeit bzw. Lösungsgeschwindigkeit des pH-sensitiven Polymers bei einem saurem pH-Wert geringer als bei einem neutralen oder alkalischem pH-Wert , da ein Anstieg des pH-Wertes bei der zu behandelnden Wunde häufig mit einem unzureichenden Heilungsverlauf korreliert.

Besonders vorteilhaft ist es im Zusammenhang mit der Erfindung, dass die Löslichkeit bzw. Lösungsgeschwindigkeit des pH-sensitiven Polymers bei einem pH-Wert von 4 geringer als bei einem pH-Wert von 8 ist.

Insbesondere ist es vorteilhaft, dass die Lösungsgeschwindigkeit des pH-sensitiven Polymers in einem Essigsäure Acetat-Puffer (10 mM Essigsäure in demineralisiertem Wasser) und bei einer Temperatur von 20° C bei einem pH-Wert von 4,0 weniger als 0,1 mg/min/g beträgt und/oder dass die Lösungsgeschwindigkeit des pH-sensitiven Polymers in einem TRIS-HCI Puffer (10 mM Tris in demineralisiertem Wasser) und bei einer Temperatur von 20° C bei einem pH-Wert von 8,0 mehr als 1 mg/min/g beträgt. Die Lösungsgeschwindigkeit wird hierbei in einem 1 I Becherglas mit einem Durchmesser von ca. 105 mm bestimmt, wobei 10,000 g Polymer in 500 ml der jeweiligen Lösung 10 min gerührt werden (Magnetrührer, 50 Umin⁻¹). Die Menge an ungelösten Polymer nach 10 min wird durch Abfiltrieren, Trocknen und Wiegen der ungelösten Substanz bestimmt. Hieraus wird die Menge an Polymer ermittelt, die sich während der 10 Minuten gelöst hat.

Besonders geeignet sind hierbei Polymere, die bei einem pH-Wert von weniger al 6,0 in wässriger Lösung nicht löslich und bei einem pH-Wert von mehr als 7,0 in einer Menge von wenigstens 1% (w/w) löslich sind.

Im Zusammenhang mit der vorliegenden Erfindung geeignete Polymere sind beispielsweise Celluloseacetatphthalat, Celluloseacetattrimellitat, Hydroxypropylmethylcelluloseacetatsuccinat, Carboxymethylethylcellulose, oxidierte regenerierte Cellulose, Polyacrylate, deren Copoylmere und Mischungen.

Bevorzugte Polymere umfassen Copolymerisate der Methacrylsäure mit Methacrylsäuremethylester, also ein Methacrylsäure-co-methylmethacrylat, bevorzugt im Verhältnis 1:1 bis 1:2. Derartige Polymere sind beispielsweise unter dem Namen Eudragit^{®} (Evonik, Darmstadt, Deutschland) erhältlich. Bevorzugte Polymerzubereitungen sind unter den Namen Eudragit^{®} S 100, Eudragit^{®} S 12,5, Eudragit FS 30 D, Eudragit^{®} L 100, Eudragit^{®} L 12,5 erhältlich. Auch Mischungen dieser Polymerzubereitungen können eingesetzt werden. Diese Polymere weisen besonders günstige pH-abhängige Lösungseigenschaften auf. Weiterhin geeignete Polymere sind Copolymere aus Methacrylsäure und Ethylacrylat, erhältlich beispielsweise als Eudragit^{®} L100-55.

Erfindungsgemäß ist in den Partikeln ein Fluoreszenzfarbstoff vorhanden. Ein Fluoreszenzfarbstoff ist eine fluoreszierende chemische Verbindung, die in der Lage ist, bei Anregung Licht wieder abzugeben. Es ist insbesondere bevorzugt, dass der Fluoreszenzfarbstoff mit einer Wellenlänge im UV-Bereich, vorzugsweise 100 bis 380 nm, vorzugsweise 200 bis 300 nm, insbesondere etwa 250 nm, insbesondere 254 nm, angeregt wird. Weiterhin wird bevorzugt, dass das wieder emittierte Licht eine Wellenlänge (Emissionswellenlänge) im sichtbaren Bereich aufweist, vorzugsweise von 400 bis 750 nm, bevorzugter von 500 bis 600 nm, insbesondere von etwa 520 nm, insbesondere 517 nm, so dass die Fluoreszenz nach einer Anregung mit bloßem Auge sichtbar ist. Fluoreszenzfarbstoffe werden oft in Klassen eingeteilt, wie z.B. Acridinfarbstoffe, Cyaninfarbstoffe, Fluoronfarbstoffe, Oxazinfarbstoffe, Phenanthridinfarbstoffe und Rhodaminfarbstoffe. Bevorzugt werden Fluoronfarbstoffe eingesetzt.

In einer bevorzugten Ausführungsform der Erfindung gehört der Fluoreszenzfarbstoff zur Gruppe der Xanthene, die mindestens eine Hydroxygruppe am Xanthenskelett tragen. Fluoreszenzfarbstoffe auf Basis eines Xanthensystems mit mindestens einer Hydroxygruppe am Xanthenskelett sind beispielsweise Eosine wie Eosin B und Eosin Y und Fluoreszenzfarbstoffe wie 6-Carboxyfluorescein (auch Carboxyfluorescein genannt), 2,7-Dichlorfluorescein und Fluorescein. Nach einer besonders bevorzugten Ausführungsform wird im Zusammenhang mit der Erfindung Fluorescein, insbesondere Carboxyfluorescein verwendet.

Bei einer Verwendung der hier vorgeschlagenen Partikel, enthaltend ein pH-sensitives Polymer und einen Fluoreszenzfarbstoff, in der Behandlung von Wunden wird so lange kein Fluoreszenzfarbstoff aus den Partikeln freigesetzt, als die Partikel unversehrt sind. Bei einer Verschiebung des pH-Wertes in der Wunde können die Partikel aufgelöst werden, so dass der Fluoreszenzfarbstoff freigesetzt wird. Sodann findet die Verdünnung des Fluoreszenzfarbstoffs statt, die bei einer geeigneten Anregung anhand der Emission sichtbaren Lichts erkannt werden kann.

Gemäß einer besonders vorteilhaften weiteren Ausführungsform der Wundauflage können die Partikel mindestens eine weitere Substanz umfassen, insbesondere eine oder mehrere therapeutische Substanzen.

Eine therapeutische Substanz kann als eine Substanz zur Behandlung oder Heilung einer Krankheit bezeichnet werden, vorzugsweise durch Erzielung einer Heilwirkung.

Beispiele für therapeutische Substanzen sind antimikrobielle Substanzen wie Antiseptika, antimikrobielle Metalle und Antibiotika, entzündungshemmende und/oder analgetische Substanzen wie nicht-steroidale entzündungshemmende Medikamente, Wachstumsfaktoren, anti-biofilmische Substanzen wie antimikrobielle Peptide (auch als AMPs bezeichnet), enzymatische Substanzen wie Trypsin, Proteaseinhibitoren wie Gewebeinhibitor von Metalloproteinasen (auch als TIMPs bezeichnet) und Mischungen davon.

Beispiele für nicht-steroidale entzündungshemmende Medikamente (auch NSAIDs genannt) sind Acetylsalicylsäure, Diclofenac, Ibuprofen, Indomethacin, Metamizol und Paracetamol. Mit derartigen Substanzen kann auch vorteilhaft eine lokale analgetische Wirkung erzielt werden.

Beispiele für Wachstumsfaktoren sind epidermaler Wachstumsfaktor (EGF), Fibroblasten-Wachstumsfaktor (FGF), insulinähnlicher Wachstumsfaktor (IGF), Keratinozyten-Wachstumsfaktor (KGF), Platelet-derived Growth Factor (PDGF), Transforming Growth Factor (TGF) oder vaskulärer endothelialer Wachstumsfaktor (VEGF).

Bevorzugt werden antimikrobielle Substanzen als therapeutische Substanz eingesetzt. Eine antimikrobielle Substanz ist ein Mittel, das dazu bestimmt ist, das Vorhandensein von Mikroorganismen, wie beispielsweise Bakterien, zu reduzieren, indem es sie abtötet oder zumindest das Wachstum ihrer Population verhindert. Zu den antimikrobiellen Substanzklassen gehören beispielsweise Desinfektionsmittel, antimikrobielle Metalle, Antiseptika und Antibiotika.

Beispiele für antimikrobielle Substanzen sind Benzalkoniumchlorid, Chlorhexidin, lod, Polyvidon-lod, Octenidin, Polyhexanid (PHMB), Silber und seine Salze, vorzugsweise in nanopartikulärer Form, Zinkoxid, vorzugsweise in nanopartikulärer Form, Beta-Lactam-Antibiotika wie Benzylpenicillin, Phenoxymethylpenicillin, Oxacillin, Ampicillin, Amoxicillin, Cefotaxim, Ceftriaxon und Cefepim, Minoglykosid-Antibiotika wie Streptomycin, Gentamicin, Kanamycin und Neomycin, Tetracyclin-Antibiotika wie Tetracyclin, Oxytetracyclin, Doxycyclin und Minocyclin, Makrolid-Antibiotika wie Erythromycin, Clarithromycin, Roxithromycin, Azithromycin, Spiramycin, Chinolon-Antibiotika wie Norfloxacin, Ciprofloxacin, Ofloxacin, Levofloxacin, Sparfloxacin und Moxifloxacin, antimikrobielle Peptide (AMPs) wie Pexiganan, Iseganan und Omigananan und Mischungen davon.

Gemäß einer besonders bevorzugten Ausführungsform handelt es sich bei der therapeutischen Substanz um ein Antibiotikum, vorzugsweise ein Chinolon-Antibiotikum, insbesondere ein Fluorchinolon-Antibiotikum. Nach einer ganz besonders bevorzugten Ausführungsform ist die therapeutische Substanz Norfloxacin, Ciprofloxacin, Ofloxacin, Levofloxacin, Sparfloxacin und/oder Moxifloxacin, insbesondere Ciprofloxacin.

Weiterhin bevorzugt betrifft die vorliegende Erfindung Wundauflagen, die Partikel enthält, welche neben dem fluoreszierenden Farbstoff und der optional vorhandenen therapeutischen Substanz zusätzlich eine oder mehrere Brönstedsäuren umfassen. Besonders bevorzugte Brönstedsäuren sind hierbei insbesondere Essigsäure, Zitronensäure, Milchsäure, Glycerinsäure, Gluconsäure, Benzoesäure, Aconitsäure, Glutarsäure, Weinsäure, Phosphorsäure, Äpfelsäure, Bernsteinsäure oder Glutaminsäure. Diese Brönstedsäuren weisen einen vorteilhaften pKs-Wert zwischen 2 und 5 auf, sind physiologisch unbedenklich und können in der Wundheilung eingesetzt werden. Besonders bevorzugt werden Zitronensäure, Benzoesäure und/oder Milchsäure als Brönstedsäuren verwendet, gegebenenfalls gemeinsam mit ihren korrespondierenden Basen. Diese Brönstedsäuren weisen eine besonders gute Gewebeverträglichkeit auf.

Gemäß dieser Ausführungsform können aus den Partikeln neben dem fluoreszierenden Farbstoff (und ggf. neben der therapeutischen Substanz) zusätzlich eine oder mehrere Brönstedsäuren an die Wunde abgegeben werden. Hierdurch wird einer Alkalisierung der Wunde entgegengewirkt und die Wundheilung unterstützt.

Vorzugsweise sind die Partikel, welche neben der oder den freisetzbaren Substanzen zusätzlich eine oder mehrere Brönstedsäuren enthalten, in einer Wundkontaktlage des Verbandes vorhanden, falls eine solche Wundkontaktlage vorgesehen ist. Eine derartige Wundkontaktschicht besitzt den Vorteil, dass anfallendes Wundexsudat unmittelbar mit in der Wundkontaktschicht enthaltener Brönstedsäure in Berührung kommt, so dass ein ungünstig alkalischer pH-Wert des anfallenden Wundexsudats sofort korrigiert werden kann.

Unter einer Brönsted-Säure wird eine Substanz verstanden, die in Reaktion mit geeigneten Lösemitteln Wasserstoffkationen (Protonen) abgeben kann. Dabei entstehen als Reaktionsprodukte ein protoniertes Lösemittelteilchen und ein sogenannter Säurerest, der zur Brönstedsäure die korrespondierende bzw. konjugierte Base darstellt. Stoffgemische, die sowohl Brönstedsäure als auch korrespondierende Base enthalten, werden als Puffersubstanzen bezeichnet. Diese Gemische zeichnen sich dadurch aus, dass sie in wässriger Lösung den pH-Wert der Lösung stabilisieren, selbst wenn zu der wässrigen Lösung Mengen einer Säure oder einer Base zugegeben werden. Eine zur wässrigen Lösung zugegebene Säure reagiert mit der in der Puffersubstanz vorhandenen Base unter Ausbildung eines neuen Teilchens der zur Base korrespondierenden Brönstedsäure. Es entstehen keine weiteren Hydroxonium- oder Hydroxidionen, so dass der pH-Wert beinahe unverändert bleibt. Diese pH-Wert-Stabilisierung funktioniert, so lange die Menge an zugegebener Säure bzw. Base die Menge an enthaltenen Basen- oder Säureteilchen nicht übersteigt. Das quantitative Ausmaß dieser Stabilisierung wird als Pufferkapazität bezeichnet.

Die gemäß dieser Ausführungsform vorgeschlagenen Wundauflage können sowohl eine Brönstedsäure alleine als auch eine Mischung aus Brönstedsäure und ihrer korrespondierenden Base enthalten. Wenn eine Brönstedsäure gemeinsam mit ihrer korrespondierenden Base eingesetzt wird, besteht das molare Verhältnis aus Brönstedsäure und ihrer korrespondierenden Base 100:1 bis 1:100, bevorzugt 10:1 bis 1:10, besonders bevorzugt 1:1. Eine äquimolare Mischung aus Brönstedsäure und ihrer korrespondierenden Base besitzt die größte pH-Wert-Stabilität gegenüber Säure- oder Basenzugabe.

In einer bevorzugten Ausführungsform enthält eine erfindungsgemäße Wundauflage eine Wundkontaktschicht. In einer weiteren bevorzugten Ausführungsform besteht eine erfindungsgemäße Wundauflage aus einer Wundkontaktschicht.

Als Wundkontaktschicht kann gemäß der vorliegenden Erfindung ein Material Verwendung finden, das keinen negativen Einfluss auf die Wundheilung ausübt. Hierbei steht eine Wundkontaktschicht bei Verwendung der erfindungsgemäßen Wundauflage in direktem Kontakt mit der Wunde. Diese Wundkontaktschicht kann einzig und allein dazu dienen, dass eine absorbierende Schicht von der zu behandelnden Wunde beabstandet wird, als auch dass diese Wundkontaktschicht weitere Funktionen in Bezug auf die Wundauflage als auch auf die zu behandelnde Wunde ausübt. Insbesondere kann eine erfindungsgemäße Wundauflage eine Wundkontaktschicht mit einer ersten Seite und einer zweiten Seite umfassen, wobei die Wundkontaktschicht eine Hydrogelmatrix, einen Polymerfilm, eine Hydrokolloidmatrix, ein Polymernetz, ein Nonwoven, ein Gewebe, ein Gewirk, ein Gestrick oder ein Adhäsiv umfasst.

Gemäß einer Weiterbildung der erfindungsgemäßen Wundauflage kann vorgesehen sein, dass die Wundkontaktschicht eine Vielzahl an Kanälen, Öffnungen oder Löcher zum Durchtritt von Flüssigkeiten umfasst. Insbesondere ist dabei vorgesehen, das die Wundkontaktschicht Kanäle aufweist, die einen Durchtritt von Wundsekret von der ersten Seite zur zweiten Seite bilden. In dieser Ausführungsform ist vorgesehen, dass die erste Seite der Wundkontaktschicht einen direkten Kontakt zu einer zu behandelnden Wunde und die zweite Seite der Wundkontaktschicht einen direkten Kontakt mit der ersten Seite der absorbierenden Schicht aufweist.

Gemäß einer Weiterbildung der Erfindung kann auch vorgesehen sein, dass die Wundkontaktschicht Kanäle, Öffnungen oder Löcher aufweist, die einen Durchmesser von 0,5 bis 5 mm aufweisen. Insbesondere weist die Wundkontaktschicht Kanäle, Öffnungen oder Löcher auf, die einen Durchmesser von 1 bis 3 mm aufweisen. Ganz besonders bevorzugt weist die Wundkontaktschicht auf der im anwendungsgerechten Zustand der Wundauflage der Wunde zugewandten, ersten Seite Öffnungen auf, die einen Durchmesser von 1 bis 3 mm aufweisen, wobei die zweite Seite der Wundkontaktschicht in direktem Kontakt mit einer absorbierenden Schicht steht.

Weiterhin bevorzugt kann die Wundkontaktschicht eine Vielzahl an Kanälen, Öffnungen oder Löcher zum Durchtritt von Flüssigkeiten aufweisen, wobei die Kanäle, Öffnungen oder Löcher an der ersten Seite der Wundkontaktschicht eine Fläche von höchstens 95 % der Fläche der ersten Seite der Wundkontaktschicht einnehmen. Hierbei ist weiterhin bevorzugt vorgesehen, dass die Kanäle, Öffnungen oder Löcher eine Fläche von höchstens 70 %, insbesondere höchstens 50 %, insbesondere höchstens 40 % und ganz besonders bevorzugt von höchstens 30 % der Fläche der ersten Seite der Wundkontaktschicht einnehmen. Ganz besonders bevorzugt weist die Wundkontaktschicht Kanäle, Öffnungen oder Löcher auf, die an der ersten Seite der Schicht eine Fläche von mindestens 5 % und höchstens 30 % der Fläche der ersten Seite der Wundkontaktschicht einnehmen. Ganz besonders weist die Wundauflage eine Wundkontaktschicht auf, die 2 bis 8 Löcher pro cm² aufweist. Hierbei kann vorgesehen sein, dass die Wundkontaktschicht ein gelochter Polymerfilm oder ein Polymernetz, insbesondere ein Polyurethanfilm oder ein Polymernetz ist.

Gemäß einer weiteren Ausführungsform der vorliegenden Erfindung kann weiterhin vorgesehen sein, dass eine erfindungsgemäße Wundauflage als Wundkontaktschicht eine Hydrokolloidmatrix umfasst. Diese Hydrokolloidmatrix kann aus einer klebenden Polymermatrix bestehen, in die Hydrokolloidpartikel dispergiert sind. Gemäß der vorliegenden Erfindung soll unter einem Hydrokolloid ein Material verstanden sein, das ein hydrophiles synthetisches oder natürliches Polymermaterial ist, das löslich oder absorbierend und/ oder quellend in Wasser ist. Vorzugsweise umfasst eine Wundkontaktschicht ein Hydrokolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum, Gelatine oder Mischungen hiervon.

Das Hydrokolloid kann sowohl in Form von Fasern als auch in Form von Partikeln und/ oder Fasern innerhalb einer Matrix vorliegen. Insbesondere kann das Hydrokolloid in Form von Partikeln in einer klebenden Polymermatrix vorliegen. Die klebende Polymermatrix umfasst dabei mindestens ein Co-Blockpolymer ausgewählt aus der Gruppe der AB-Diblock-Copolymere und/ oder ABA-Triblock-Copolymere, das aus den Monomeren Styrol, Butadien und Isopren aufgebaut ist. Der Anteil an Hydrokolloidpartikel in der Wundkontaktschicht kann vorzugsweise 10 bis 70 Gew.-% bezogen auf das Gesamtgewicht der Wundkontaktschicht aufweisen. Eine solche Zusammensetzung ist zum Beispiel mit der EP 1 007 597 B1 bekannt.

Gemäß einer weiteren Ausführungsform der vorliegenden Erfindung kann weiterhin vorgesehen sein, dass eine erfindungsgemäße Wundauflage als Wundkontaktschicht eine wasserhaltige Hydrogelmatrix umfasst.

Geeignete Hydrogelmatrices können gelbildende Polymere umfassen wie beispielsweise Methylcellulose, Ethylcellulose, Hydroxyethylcellulose, Hydroxypropylmethylcellulose, Carboxymethylcellulose und ihr Natriumsalz, Hydroxypropylcellulose, Polyacrylate, Polymere von Vinylalkoholen, Vinylestern, Vinylethern und von Carboxyvinylmonomeren, Polyvinylpyrrolidon, Polyacrylamide, Polyethylenglykole, Polypropylenglykole, Copolymere aus Polyethylenglykol und Polypropylenglykol, Polystyrene, Polymere der Acrylamidomethylpropansulfonsäure und ihrer Salze, Alginate, Hyaluronsäure, Polyurethane sowie deren Mischungen umfassen. Geeignete Hydrogele sind beispielsweise in den veröffentlichten Patentanmeldungen WO00/045864 und DE102005035879 beschrieben.

In einer bevorzugten Ausführungsform enthält eine erfindungsgemäße Wundauflage eine absorbierende Schicht. In einer weiteren bevorzugten Ausführungsform besteht eine erfindungsgemäße Wundauflage aus einer absorbierenden Schicht. Unter einer absorbierenden Schicht wird eine Schicht verstanden, die in der Lage ist, Flüssigkeit, bevorzugt Wundexsudat, aufzunehmen und zu speichern. Bevorzugt verwendet werden Lagen, die ein Flüssigkeitsaufnahmevermögen von mindestens 1g/g, stärker bevorzugt von mindestens 2g/g, stärker bevorzugt von mindestens 5g/g, besonders bevorzugt von mindestens 10 g/g, gemessen nach DIN-EN 13726-1 (2002).

Eine absorbierende Schicht kann einen Schaumstoff, ein Gewebe, ein Gewirk, ein Gestrick, einen Vliesstoff oder ein Fasermaterial enthalten.

Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz. So kann die Dichte eines Schaumstoffes, bevorzugt eines Polyurethanschaumstoffes, beispielsweise zwischen 10 und 110 kg/m³, bevorzugt 15 bis 100 kg/m³, mehr bevorzugt 20 bis 95 kg/m³ aufweisen.

Gewöhnlicherweise werden unter Gewebe Webereierzeugnisse verstanden. Hierzu zählen beispielsweise Tuch, Samt und sonstige textile Flächengebilde aus speziellen Fadenanordnungen, welche im Wesentlichen senkrecht zueinander sind. Die Fäden in Längsrichtung werden Kettfäden genannt und die Fäden in Querrichtung heißen Schussfäden. Um eine ausreichende Festigkeit des Gewebes zu erreichen, müssen die Kett- und Schussfäden dicht miteinander verwoben werden und zeigen deshalb ein geschlossenes Erscheinungsbild.

Gewirke sind auch unter dem Begriff Gewirk oder Wirkwaren bekannt. Gewirke gehören zu den Maschenwaren und sind aus Fadensystemen durch Maschenbildung meist maschinell hergestellte Stoffe.

Unter einem Vliesstoff soll ein Flächen- oder Raumgebilde aus gerichtet angeordneten oder wahllos zueinander befindlichen Fasern verstanden werden, welche mechanisch und/oder thermisch und/oder chemisch verfestigt wurden. Die Vliesstoffe (im Englischem auch "nonwoven") sind wesentlich verschieden von Geweben, Gestricken und Gewirken.

Die Vliesstoffe der vorliegenden Erfindung können Fasern natürlichen oder synthetischen Ursprungs oder Gemische davon enthalten. Zu den Fasern natürlichen Ursprungs zählen z.B. Seide, Cellulose, Baumwolle und Wolle. Die Fasern synthetischen Ursprungs umfassen die synthetischen Polymere (Kunstfasern) wie Viskose, Polyacrylate, Polyamide, Polyimide, Polyamidimide, Polyurethane, Polyester (insbesondere Polyethylenterephthalate und Polybutylenterephthalate), Polyetherester, Polyether, Polyacrylnitrile, Polyalkene (insbesondere Polyethylene und Polypropylene) Polyurethane und Polytetrafluorethylene.

Das Fasermaterial ist bevorzugt ein hydrophiles Fasermaterial. Hierbei können Fasern aus Cellulose, bevorzugt wasserunlösliche Fasern aus Cellulose, insbesondere weitgehend delignifizierte technische Zellstofffasern, insbesondere Holzstofffasern verwendet werden. Insbesondere können Fasern mit einer Faserlänge von < 5 mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten. Es kann auch vorgesehen sein eine Fasermischung aus Cellulose-, regenerierter Cellulose-, Carboxymethylcellulose-, Carboxyethylcellulose-, Hydroxymethylcellulose- oder Hydroxyethylcellulose-Fasern und Fasern aus Polyethylen, Polypropylen oder Polyester zu verwenden.

Die absorbierende Schicht kann zusätzlich ein Gemisch aus Polyacrylatpartikeln enthalten, wobei die Polyacrylatpartikel ein vernetztes und/oder quervernetztes Polyacrylat umfassen. Es kann jedoch auch vorgesehen sein, dass zwischen der absorbierenden Schicht und der Wundkontaktschicht eine Übergangsschicht angeordnet ist. Eine derartige Übergangsschicht kann dadurch entstehen, dass Teile der Wundkontaktschicht mit Teilen der absorbierenden Schicht vermischt werden. So entsteht eine besonders stabile Verbindung zwischen Wundkontaktschicht und absorbierender Schicht. Eine Übergangsschicht kann auch in einer Lage einer weiteren flüssigkeitsdurchlässigen Materialschicht sein, die sowohl mit der Wundkontaktschicht als auch mit der absorbierenden Schicht verbunden ist. In diesem Fall sorgt die Übergangsschicht für einen schnellen und gleichmäßigen Transport anfallenden Wundexsudats von der Wundoberfläche zur absorbierenden Schicht.

Gemäß einem weiteren weiterführenden Gedanken der vorliegenden Erfindung, ist auch eine Wundauflage Gegenstand der vorliegenden Erfindung, die zwischen der Hydrogelmatrix und dem hydrophilen Polymerschaum eine Barriereschicht aufweist. Eine solche Barriereschicht kann beispielsweise einen Polymerfilm umfassen, der mit Öffnungen versehen ist.

Im Zusammenhang mit den vorstehend beschriebenen Wundkontaktschichten oder den absorbierenden Schichten wird bevorzugt vorgeschlagen, dass die Partikel in der Wundkontaktschicht und/oder in der absorbierenden Schicht enthalten sind.

Weiterhin kann eine erfindungsgemäße Wundauflage eine Trägerschicht umfassen. Diese Trägerschicht kann aus verschiedenen Materialien bestehen. Üblicherweise werden in Wundauflagen textile Trägermaterialien, Nonwoven, Polymerfilme oder Polymerschäume eingesetzt.

Als Trägerschicht einer erfindungsgemäßen Wundauflage können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässigen und wasserdampfdurchlässigen Polyurethanfilm oder einen wasserundurchlässigen und wasserdampfdurchlässigen Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 750 g/ m²/ 24 Std., insbesondere mindestens 1000 g/ m2/ 24 Std. und ganz besonders bevorzugt mindestens 2000 g/ m²/ 24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m² zusammen mit dem Film eine Wasserdampfdurchlässigkeit von mindestens 800 g/ m²/ 24 Std. und vorzugsweise von mindestens 1000 g/ m²/ 24 Std. (gemessen nach DIN EN 13726) aufweisen.

Bezüglich der inneren Struktur der Partikel werden im Zusammenhang mit der Erfindung im Folgenden besonders vorteilhafte Varianten näher beschrieben:
Zum einen ist denkbar, dass der Fluoreszenzfarbstoff sowie optional weitere für die Wundheilung vorteilhafte Substanzen in eine in den Partikeln vorhandene Matrix aus dem pH-sensitivem Polymer eingebettet, also dispergiert, vorliegt. Mikrosphärulen sind Partikel, bei denen der Fluoreszenzfarbstoff in einer Polymermatrix ohne Ausbildung einer gesonderten Kapselwand eingebettet ist. In einer bevorzugten Ausführungsform enthält eine Wundauflage Partikel, die eine aus einem pH-sensitiven Polymer gebildete Matrix umfassen, in die ein Fluoreszenzfarbstoff, insbesondere Carboxyfluorescein, eingebettet ist.

Alternativ kann der Fluoreszenzfarbstoff sowie die weiteren optional vorhandenen Substanzen von dem pH-sensitivem Polymer umhüllt sein. Dies kann insbesondere dadurch erreicht werden, dass die Partikel einen Kern aufweisen, welcher den Fluoreszenzfarbstoff enthält und welcher von dem pH-sensitiven Polymer umhüllt ist, wobei in dem Kern optional zusätzlich eine oder mehrere Trägersubstanzen vorhanden sein können.

Nanopartikel sind kolloidale Feststoffsysteme mit einem Durchmesser von 50-1000 nm. Es wird zwischen Nanokapseln und Nanosphärulen unterschieden.

Nanokapseln sind verfestigte mizellare Systeme, verfestigte Mikroemulsionen oder umhüllte kolloidale Feststoffsysteme.

Nanosphärulen werden auch als Nanopellets bezeichnet und sind kolloidale Teilchen, bei denen ein Stoff in eine Polymermatrix eingebettet ist.

Bevorzugt können auch Partikel mit einem Äquivalentdurchmesser von 5 µm-300 µm vorteilhaft verwendet werden. Derartige Mikropartikel sind in der Lage, auch in feine Öffnungen des Materials der Lage, in der die Partikel eingebracht werden, einzudringen und so gleichmäßig in der jeweiligen Lage verteilt vorzuliegen. Aufgrund des geringen Äquivalenzdurchmessers wird eine hohe Oberfläche der Gesamtheit von Polymerpartikeln erreicht, so dass anfallendes Wundexsudat unmittelbar in innigen Kontakt mit den Fluoreszenzfarbstoff-haltigen Partikeln kommt, so dass bei Bedarf sofort eine ausreichende Menge an Fluoreszenzfarbstoff und ggf. weiteren Substanzen freigesetzt werden kann.

Der Äquivalentdurchmesser kann mittels Laserbeugung bestimmt werden, beispielsweise mit einem Mastersizer 2000 der Firma Malvern. Die Auswertung erfolgt bevorzugt nach der Fraunhofer-Methode.

Geeignete Partikel können nach verschiedenen Verfahren hergestellt werden. Grundsätzlich sind dem Fachmann zahlreiche Verfahren zur Herstellung von Partikeln bekannt, in denen eine Substanz mit einem geeigneten polymeren Material kombiniert wird. So wird beispielsweise auf das Lehrbuch der Pharmazeutischen Technologie der Autoren Bauer, Frömming und Führer hingewiesen, welches 1999 in der sechsten Auflage bei der Wissenschaftlichen Verlagsgesellschaft Stuttgart erschienen ist.

Einige geeignete Herstellungsverfahren werden im Folgenden beispielhaft dargestellt.

Bei den Lösemittelextraktions/-evaporationsverfahren werden Emulsionströpfchen einer organischen Polymerlösung, in die ein Fluoreszenzfarbstoff und ggf. weitere im Zusammenhang mit der Erfindung zur Unterstützung der Wundheilung vorgeschlagene Substanzen auf verschiedene Weise eingearbeitet sein können, in eine Flüssigkeit eingebracht, in der das organische Lösemittel der dispersen Phase löslich ist und dadurch aus den Tröpfchen extrahiert wird. Der Fluoreszenzfarbstoff und ggf. weitere Substanzen können entweder gemeinsam mit dem Polymer in der dispersen Phase gelöst oder in fein pulverisierter Form in dieser dispergiert werden. Ebenso können wässrige Lösungen eines Fluoreszenzfarbstoffes und ggf. weiterer Substanzen in der dispersen Phase emulgiert werden, wodurch sich mit der kontinuierlichen, äußeren Phase eine Doppelemulsion bildet. Die Tröpfchenbildung erfolgt mit Hilfe von Rührwerkzeugen oder statischen Mischern. Dieser Prozessschritt bestimmt maßgeblich die mittlere Partikelgröße und die Partikelgrößenverteilung des jeweiligen Endproduktes. Im einfachsten Fall wird die äußere Phase in einem Mischgefäß vorgelegt und bei ausreichender Rührgeschwindigkeit die farbstoffhaltige Polymerlösung zugegeben. Auch die umgekehrte Vorgehensweise, also die Zugabe der äußeren Phase in die gerührte Polymerlösung ist möglich, wobei in diesem Fall die Tröpfchenbildung unter Phaseninversion erfolgt. Durch Entzug des Polymer-Lösemittels werden die gebildeten Tröpfchen ausgehärtet und in Mikrosphärulen überführt. Bei ausreichender Löslichkeit des verwendeten Lösemittels in der äußeren Phase oder bei deren ausreichend hohem Überschuss erfolgt der Lösemittelentzug aus den Emulsionströpfchen allein durch einen Extraktionsprozess. Wenn das Verteilungsgleichgewicht jedoch nicht genügend weit auf der Seite des Extraktionsmittels liegt, muss der Vorgang durch kontinuierlichen Entzug des Polymerlösemittels beispielsweise durch Evaporation oder Dialyse unterstützt werden. Im Hinblick auf eine hohe Einschlusseffizienz werden vorzugsweise solche Extraktionsflüssigkeiten verwendet, in denen der Fluoreszenzfarbstoff (sowie die ggf. vorhandenen weiteren Substanzen) nicht oder nur schwerlöslich ist. Die Geschwindigkeit des Lösungsmittelentzugs ist ein wesentlicher Einflussfaktor, der genutzt werden kann, um die innere Struktur und mit dieser das Freisetzungsverhalten der Mikropartikel gezielt zu steuern. Nach dem Extraktions-/Evaporationsprozess werden die entstandenen Mikropartikel abfiltriert oder abzentrifugiert, gewaschen und unter materialschonenden Bedingungen getrocknet.

Beim Phasenseparationsverfahren wird der Fluoreszenzfarbstoff (und ggf. die weiteren Substanzen) in wässriger Lösung oder als mikronisierte Festsubstanz in einer organischen Polymerlösung, beispielsweise Dichlormethan, Ethylacetat, Acetonitril emulgiert bzw. suspendiert. Durch Zugabe eines Phasentrennmittels, beispielsweise Silikonöl, kommt es zur Koazervation des Polymers. Durch Adsorption des Koazervates an die Oberfläche der wässrigen Tröpfchen bzw. der festen Farbstoffpartikel, werden diese von einer zunächst noch sehr weichen Polymerhülle überzogen. Durch Auswaschen des Phasentrennmittels durch ein Nichtlösemittel für das Polymer, beispielsweise Hexan oder Heptan, wird die Hülle gehärtet, wonach die entstandenen Mikrokapseln abgetrennt werden können.

Beim Sprühtrocknungsverfahren wird eine gemeinsame Lösung von Fluoreszenzfarbstoff (und ggf. weiterer Substanzen), in einem organischen Lösemittel oder eine Suspension des Farbstoffes in der organischen Polymerlösung in einen heißen Luftstrom hinein zerstäubt. Das trockene Produkt wird durch einen Zyklon aus dem Luftstrom abgetrennt. Mit diesem Verfahren werden Partikel erhalten, bei denen der Fluoreszenzfarbstoff in einer Matrix aus pH-sensitivem Polymer eingebettet sind. Derartige Partikel können besonders kostengünstig hergestellt werden.

In einem weiteren geeigneten Herstellungsverfahren wird Fluoreszenzfarbstoff (und ggf. weitere Substanzen) in Pulverform in einen Dragierkessel gegeben und mit einer geeigneten Polymerlösung oder Polymerdispersion besprüht. Es entstehen Mikrokapseln.

In einem weiteren geeigneten Herstellungsverfahren wird der Fluoreszenzfarbstoff (und ggf. weitere Substanzen) in Pulverform in der Wirbelschicht mit einer geeigneten Polymerlösung oder Polymerdispersion besprüht. Ein besonders geeignetes Verfahren ist das Wurster-Verfahren.

In einem weiteren geeigneten Verfahren wird eine Puffersubstanz zu einer Lösung eines geeigneten Monomers gegeben und das Monomer zur Polymerisation veranlasst. Dabei entstehen Partikel, in denen der Fluoreszenzfarbstoff (und ggf. weitere Substanzen) in eine polymere Matrix eingebettet sind.

Im Rahmen der vorliegenden Erfindung können eingesetzte Partikel ein Massenverhältnis von Farbstoff bzw. der Gesamtheit von freisetzbaren Substanzen zu Polymer zwischen 100:1 und 1:100 aufweisen, bevorzugt zwischen 10:1 und 1:10, besonders bevorzugt zwischen 10:1 und 1:1. Aus derartigen Partikeln wird der enthaltene Farbstoff bzw. auch die weiteren freisetzbaren Substanzen bei Kontakt mit Wundexsudat mit erhöhtem pH-Wert mit einer besonders vorteilhaften Geschwindigkeit freigesetzt.

Beschrieben (nicht erfindungsgemäß ) werden auch Partikel, die ein pH-sensitives Polymer und einen Fluoreszenzfarbstoff umfassen, zur Verwendung in der Behandlung von Wunden, insbesondere infizierter Wunden und/oder chronischer Wunden.

Bei einer erfindungsgemäßen Wundauflage können die Partikel mit dem Fluoreszenzfarbstoff in einer Wundkontaktschicht enthalten sein. Bei einer derartigen Ausführung erfolgt bei einer pH-Veränderung in der Wunde eine besonders rasche Freisetzung des Farbstoffes. Die Wundkontaktschicht kann ein poröses, flüssigkeitsdurchlässiges Material umfassen. Dieses Material kann zunächst mit einer Suspension, welche die Partikel enthält, imprägniert und anschließend vollständig getrocknet werden, so dass die Partikel mit dem Farbstoff und dem pH-sensitiven Polymer vollständig im entsprechenden Material enthalten sind.

Falls es sich bei der Wundkontaktschicht um eine halbfeste Zubereitung wie beispielsweise eine Salbe, Creme, Paste oder ein Gel, bevorzugt ein Hydrogel handelt, kann eine Suspension aus den Partikeln direkt in die vorgefertigte halbfeste Zubereitung eingearbeitet werden, beispielsweise durch Verrühren oder Verreiben. Eine Suspension aus den erfindungsgemäßen Partikeln, kann auch in einem der Bestandteile der halbfesten Zubereitung gelöst oder dispergiert werden und anschließend mit den übrigen notwendigen Bestandteilen zur fertigen halbfesten Zubereitung weiterverarbeitet werden.

Eine derartige Wundkontaktschicht kann alleine als primäre Wundauflage verwendet werden, gemeinsam mit darauf aufgebrachten sekundären Wundauflagen oder als Schicht in einer mehrschichtigen Wundauflage enthalten sein.

Eine erfindungsgemäße Wundauflage kann eine absorbierende Schicht enthalten. Diese absorbierende Schicht kann zunächst mit einer Partikel-Suspension imprägniert und anschließend vollständig getrocknet werden, so dass die Partikel vollständig im entsprechenden Material enthalten sind.

Alternativ kann das Material mit einer wässrigen Suspension aus den Partikeln und dem pH-sensitivem Polymer imprägniert oder besprüht werden. Anschließend wird das Material wiederum vollständig getrocknet, so dass die Partikel im Material verbleiben.

Eine derartige absorbierende Schicht kann große Mengen an Fluoreszenzfarbstoff enthalten.

Eine derartige absorbierende Schicht kann alleine als primäre Wundauflage verwendet werden, gemeinsam mit darauf aufgebrachten sekundären Wundauflagen oder als Schicht in einer mehrschichtigen Wundauflage enthalten sein.

### Beispiele:

### Beispiel 1: Eine Lösung aus 6-Carboxyfluorescein kann hergestellt werden, indem

Carboxyfluorescein (1877 mg), Natriumchlorid (58 mg) und Natriumhydroxid (540 mg) sowie eine steril filtrierte HEPES-Pufferlösung (0,01 M) in Wasser zugesetzt werden, um 100 ml Fluoreszenzfarbstofflösung zu erhalten. Es ist auch möglich entsprechende organische Lösungen anzusetzen, beispielsweise in DMSO. 6-Carboxyfluorescein ist kommerziell auch als Mischung mit seinem Isomer 5-Carboxyfluorescein erhältlich und wird dann als 5(6)-Carboxyfluorescein bezeichnet.

Als pH-sensitives Polymer kann beispielsweise handelsübliches EUDRAGIT S 12,5 (Evonik) verwendet werden. EUDRAGIT S 12,5 besteht aus anionischen Copolymeren auf Basis von Methacrylsäure und Methyl Methacrylat (Methacrylsäure-co-methylmethacrylat-Polymer). Mischungen aus 6-Carboxyfluorescein und EUDRAGIT S 12,5 können zur Herstellung von erfindungsgemäßen Partikeln eingesetzt werden, beispielsweise unter Zuhilfenahme der vorliegend erwähnten Verfahren.

### Beispiel 2: Auf einem Hydrogel basierende Wundkontaktschicht

Für die Herstellung des Hydrogels wurden die wässrigen Flüssigkeiten A und B wie folgt hergestellt.

### Wässrige Flüssigkeit A:

Natriumchlorid (6,24 g; 0,107 mol) wurde sterilisiertem und demineralisiertem Wasser (990 ml) zugesetzt und fünf Minuten lang bei 23°C zu gerührt, um Lösung 1 zu bilden. Lösung 1 (767,5 g) und Jeffamin-Mix (50 Gew.-% Jeffamin und 50 Gew.-% Wasser; 132,5 g) wurden bei 23°C unter Rühren für fünf Minuten gemischt, um die wässrige Flüssigkeit A zu bilden (900 g).

### Wässrige Flüssigkeit B:

Wässrige Lösung B ist ein aliphatisches Isocyanatpräpolymer in Wasser (Isophorondiisocyanat in Wasser, erhältlich unter dem Namen Aquapol von Carpenter; 900 g).

Die Herstellung des Hydrogels erfolgte in der Pilotgießanlage B100. Die vorstehend vorbereiteten wässrigen Flüssigkeiten A und B wurden in die beiden Kartuschen der Gießanlage gefüllt. Um eine genaue Dosierung und Reproduzierbarkeit zu gewährleisten, wurde die Flüssigkeiten nach dem Befüllen für 12 Stunden in den Kartuschen belassen. Hierdurch können Ungenauigkeiten, z.B. durch Blasenbildung, vermieden werden. Der Inhalt der Kartuschen wurde über eine Pumpe durch Schläuche in einer Mischkammer übertragen, wobei ein Verhältnis von wässriger Flüssigkeit B zu wässriger Flüssigkeit A auf 1,5 : 1 eingestellt wurde. In der Mischkammer wurden die beiden Komponenten von einem Mischer gerührt und die Reaktion konnte stattfinden. Das noch nicht vollständig polymerisierte Hydrogel wurde aus der Mischkammer ausgelassen und konnte nach dem Gießen in Platten vollständig aushärten. Der pH-Wert des Gels wurde durch Waschen mit 0,1 M HCI auf einen Wert von etwa 6,5 bis 6,8 eingestellt.

Eine 10 cm x 10 cm messendes Hydrogellage (Dicke 0,5 cm) wurde mit 10 ml einer wässrigen Partikel-Suspension besprüht oder getränkt. Die mittels eines Sprühtrocknungsverfahrens gewonnenen Partikel enthielten 6-Carboxyfluorescein sowie Eudragit^{®} S 12,5. Die Fluoreszenz-Polymerpartikel lagerten sich in die Hydrogelstruktur ein.

### Beispiel 3: Imprägnierte absorbierende Schicht

Auf einen offenzelligen weißen Polyurethan-Schaum wurde pro Gramm Schaummaterial 15 ml einer wässrigen Partikel-Suspension (erhältlich beispielsweise durch eines der in der der vorliegenden Anmeldung beschriebenen Verfahren) aufgebracht. Nach der Imprägnierung wurde eine vollständige Trocknung des Polyurethan-Schaums vorgenommen. Nach vollständiger Trocknung verbleibt ein mit den Partikeln imprägnierter Schaum.

### Beispiel 4: Herstellung von Mikropartikeln mittels Sprühtrocknung

Es wurden Mikropartikel aus 6-Carboxyfluorescein und EUDRAGIT S 12,5 hergestellt. Dazu wurden jeweils 300ml einer Dispersion hergestellt, die anschließend mit Hilfe eines Büchi 190 Mini Spray Dryers sprühgetrocknet wurden.

Die Sprühtrocknung erfolgte mit einem Büchi 190 Mini Spray Dryer (Büchi Laboratoriums-Technik, Eislingen Deutschland). Als Trocknungstemperatur wurde 95°C gewählt. Die Pumpleistung, mit der die Dispersion versprüht wurde, lag bei ca. 5 ml/min. Als Aspiratorleistung wurde eine Einstellung von 50% gewählt. Die Lufttemperatur betrug am Auslass 48°C.

Zur weiteren Verarbeitung wurde der erhaltene Rückstand entweder in Wasser suspendiert, so dass eine Suspension erhalten wurde, oder nach erneutem Verreiben in einer Reibeschale direkt in ein geeignetes Medium eingearbeitet.

### Beispiel 5: Mikropartikel in einer Wundkontaktschicht

Ein 10 cm x 10 cm messendes Polyamidgewirk mit einem Flächengewicht von 85 g/m² wird mit 1,418 g einer 30 %-igen Suspension aus gemäß Beispiel 4 erhaltenen Farbstoff-Polymermikropartikeln mit einem Polymergehalt von 10% behandelt. Nach vollständiger Trocknung verbleiben die Fluoreszenz-Polymerpartikel in dem Polyamidgewirk.

## Patentansprüche

1. Wundauflage enthaltend Partikel, welche ein pH-sensitives Polymer und einen Fluoreszenzfarbstoff umfassen, **dadurch gekennzeichnet, dass** die Löslichkeit oder Lösungsgeschwindigkeit des pH-sensitiven Polymers bei einem saurem pH-Wert geringer als bei einem neutralen oder alkalischem pH-Wert ist, weiter **dadurch gekennzeichnet, dass** die Partikel einen Durchmesser von 50 nm bis 1000 µm, bevorzugt 5 µm bis 300 µm, aufweisen.

2. Wundauflage nach Anspruch 1 , **dadurch gekennzeichnet, dass** die Löslichkeit oder Lösungsgeschwindigkeit des pH-sensitiven Polymers bei einem pH-Wert von 4 geringer als bei einem pH-Wert von 8 ist.

3. Wundauflage nach einem der vorangehenden Ansprüche, **dadurch gekennzeichnet, dass** die Lösungsgeschwindigkeit des pH-sensitiven Polymers in einem Essigsäure Acetat-Puffer (10 mM Essigsäure in demineralisiertem Wasser) und bei einer Temperatur von 20° C bei einem pH-Wert von 4,0 weniger als 0,1 mg/min/g beträgt und/oder dass die Lösungsgeschwindigkeit des pH-sensitiven Polymers in einem TRIS-HCI Puffer (10 mM Tris in demineralisiertem Wasser) und bei einer Temperatur von 20° C bei einem pH-Wert von 8,0 mehr als 1 mg/min/g beträgt, wobei die Lösungsgeschwindigkeit wie in der Beschreibung angegeben bestimmt wird.

4. Wundauflage nach einem der vorangehenden Ansprüche, **dadurch gekennzeichnet, dass** es sich bei dem pH-sensitiven Polymer um ein Methacrylsäure-co-methylmethacrylat-Polymer handelt.

5. Wundauflage nach einem der vorangehenden Ansprüche, **dadurch gekennzeichnet, dass** die Partikel mindestens eine weitere Substanz umfassen, insbesondere eine oder mehrere therapeutische Substanzen.

6. Wundauflage nach Anspruch 5, **dadurch gekennzeichnet, dass** die mindestens eine weitere Substanz Norfloxacin, Ciprofloxacin, Ofloxacin, Levofloxacin, Sparfloxacin und/oder Moxifloxacin umfasst.

7. Wundauflage nach Anspruch 5, **dadurch gekennzeichnet, dass** die mindestens eine weitere Substanz Benzalkoniumchlorid, Chlorhexidin, Iod, Polyvidon-lod, Octenidin, Polyhexanid, Silber, Zinkoxid oder antimikrobielle Peptide umfasst.

8. Wundauflage nach einem der vorangehenden Ansprüche, **dadurch gekennzeichnet, dass** die Partikel zusätzlich eine oder mehrere Brönstedsäuren umfassen, insbesondere Essigsäure, Zitronensäure, Milchsäure, Glycerinsäure, Gluconsäure, Benzoesäure, Aconitsäure, Glutarsäure, Weinsäure, Phosphorsäure, Äpfelsäure, Bernsteinsäure oder Glutaminsäure.

9. Wundauflage nach einem der vorangehenden Ansprüche, **dadurch gekennzeichnet, dass** der Fluoreszenzfarbstoff in eine in den Partikeln vorhandene Matrix aus dem pH-sensitivem Polymer eingebettet ist oder von dem pH-sensitivem Polymer umhüllt ist.

10. Wundauflage nach einem der vorangehenden Ansprüche, **dadurch gekennzeichnet, dass** die Partikel einen Kern aufweisen, welcher den Fluoreszenzfarbstoff enthält und welcher von dem pH-sensitiven Polymer umhüllt ist, wobei in dem Kern optional zusätzlich eine oder mehrere Trägersubstanzen vorhanden sein können.

11. Wundauflage nach einem der vorangehenden Ansprüche, **dadurch gekennzeichnet, dass** sie eine Wundkontaktschicht enthält oder aus einer Wundkontaktschicht besteht.

12. Wundauflage nach einem der vorangehenden Ansprüche, **dadurch gekennzeichnet, dass** sie eine absorbierende Schicht enthält oder aus einer absorbierenden Schicht besteht.

13. Wundauflage nach Anspruch 11 oder 12, **dadurch gekennzeichnet, dass** die Partikel in der Wundkontaktschicht und/oder in der absorbierenden Schicht enthalten ist.

## Claims

1. Wound dressing comprising particles that comprise a pH-sensitive polymer and a fluorescent dye, **characterized in that** the solubility or rate of dissolution of the pH-sensitive polymer at an acidic pH is lower than at a neutral or alkaline pH, further **characterized in that** the particles have a diameter of 50 nm to 1000 µm, preferably 5 µm to 300 µm.

2. Wound dressing according to Claim 1, **characterized in that** the solubility or rate of dissolution of the pH-sensitive polymer at pH 4 is lower than at pH 8.

3. Wound dressing according to either of the preceding claims, **characterized in that** the rate of dissolution of the pH-sensitive polymer in an acetic acid-acetate buffer (10 mM acetic acid in demineralized water) and at a temperature of 20°C at pH 4.0 is less than 0.1 mg/min/g and/or **in that** the rate of dissolution of the pH-sensitive polymer in a TRIS-HCl buffer (10 mM Tris in demineralized water) and at a temperature of 20°C at pH 8.0 is more than 1 mg/min/g, the rate of dissolution being determined as specified in the description.

4. Wound dressing according to any of the preceding claims, **characterized in that** the pH-sensitive polymer is a methacrylic acid-co-methyl methacrylate polymer.

5. Wound dressing according to any of the preceding claims, **characterized in that** the particles comprise at least one further substance, in particular one or more therapeutic substances.

6. Wound dressing according to Claim 5, **characterized in that** the at least one further substance is norfloxacin, ciprofloxacin, ofloxacin, levofloxacin, sparfloxacin and/or moxifloxacin.

7. Wound dressing according to Claim 5, **characterized in that** the at least one further substance is benzalkonium chloride, chlorhexidine, iodine, polyvidone-iodine, octenidine, polyhexanide, silver, zinc oxide or antimicrobial peptides.

8. Wound dressing according to any of the preceding claims, **characterized in that** the particles additionally comprise one or more Brønsted acids, in particular acetic acid, citric acid, lactic acid, glyceric acid, gluconic acid, benzoic acid, aconitic acid, glutaric acid, tartaric acid, phosphoric acid, malic acid, succinic acid or glutamic acid.

9. Wound dressing according to any of the preceding claims, **characterized in that** the fluorescent dye is embedded in a matrix of the pH-sensitive polymer present in the particles or is encased by the pH-sensitive polymer.

10. Wound dressing according to any of the preceding claims, **characterized in that** the particles have a core that comprises the fluorescent dye and that is encased by the pH-sensitive polymer, wherein one or more vehicle substances may optionally be additionally present in the core.

11. Wound dressing according to any of the preceding claims, **characterized in that** it comprises or consists of a wound-contact layer.

12. Wound dressing according to any of the preceding claims, **characterized in that** it comprises or consists of an absorbent layer.

13. Wound dressing according to Claim 11 or 12, **characterized in that** the particles are contained in the wound-contact layer and/or in the absorbent layer.

## Revendications

1. Pansement contenant des particules qui comprennent un polymère sensible au pH et un colorant fluorescent, **caractérisé en ce que** la solubilité ou vitesse de dissolution du polymère sensible au pH est plus faible à un pH acide qu'à un pH neutre ou alcalin, en outre **caractérisé en ce que** les particules présentent un diamètre de 50 nm à 1 000 µm, de préférence 5 µm à 300 µm.

2. Pansement selon la revendication 1, **caractérisé en ce que** la solubilité ou vitesse de dissolution du polymère sensible au pH est plus faible à un pH de 4 qu'à un pH de 8.

3. Pansement selon l'une quelconque des revendications précédentes, **caractérisé en ce que** la vitesse de dissolution du polymère sensible au pH dans un tampon acide acétique-acétate (acide acétique 10 mM dans de l'eau déminéralisée) et à une température de 20 °C à un pH de 4,0 est de moins de 0,1 mg/min/g et/ou **en ce que** la vitesse de dissolution du polymère sensible au pH dans un tampon TRIS-HCL (Tris 10 mM dans de l'eau déminéralisée) et à une température de 20 °C à un pH de 8,0 est de plus de 1 mg/min/g, la vitesse de dissolution étant déterminée comme indiqué dans la description.

4. Pansement selon l'une quelconque des revendications précédentes, **caractérisé en ce que** pour ce qui est du polymère sensible au pH il s'agit d'un copolymère acide méthacrylique/méthacrylate de méthyle.

5. Pansement selon l'une quelconque des revendications précédentes, **caractérisé en ce que** les particules comprennent au moins une substance supplémentaire, en particulier une ou plusieurs substances thérapeutiques.

6. Pansement selon la revendication 5, **caractérisé en ce que** ladite au moins une substance supplémentaire comprend la norfloxacine, la ciprofloxacine, l'ofloxacine, la lévofloxacine, la sparfloxacine et/ou la moxifloxacine.

7. Pansement selon la revendication 5, **caractérisé en ce que** ladite au moins une substance supplémentaire comprend le chlorure de benzalkonium, la chlorhexidine, l'iode, la polyvidone iodée, l'octénidine, le polyhexanide, l'argent, l'oxyde de zinc ou des peptides antimicrobiens.

8. Pansement selon l'une quelconque des revendications précédentes, **caractérisé en ce que** les particules comprennent en outre un ou plusieurs acides de Brönsted, en particulier de l'acide acétique, citrique, lactique, glycérique, gluconique, benzoïque, aconitique, glutarique, d-tartrique, phosphorique, malique, succinique ou glutamique.

9. Pansement selon l'une quelconque des revendications précédentes, **caractérisé en ce que** le colorant fluorescent est noyé dans une matrice à base du polymère sensible au pH, présente dans les particules, ou est enrobé par le polymère sensible au pH.

10. Pansement selon l'une quelconque des revendications précédentes, **caractérisé en ce que** les particules comportent un noyau qui contient le colorant fluorescent et qui est enrobé par le polymère sensible au pH, en option une ou plusieurs substances véhicule pouvant en outre être présentes dans le noyau.

11. Pansement selon l'une quelconque des revendications précédentes, **caractérisé en ce qu'**il contient une couche venant en contact avec la plaie ou consiste en une couche venant en contact avec la plaie.

12. Pansement selon l'une quelconque des revendications précédentes, **caractérisé en ce qu'**il contient une couche absorbante ou consiste en une couche absorbante.

13. Pansement selon la revendication 11 ou 12, **caractérisé en ce que** la particule est contenue dans la couche venant en contact avec la plaie et/ou dans la couche absorbante.
